(19) **Europäisches Patentamt / European Patent Office / Office européen des brevets**

(11) **EP 3 977 465 B1**

(12) **EUROPEAN PATENT SPECIFICATION**

(45) Date of publication and mention
of the grant of the patent:
**27.08.2025 Bulletin 2025/35**

(21) Application number: **20728787.1**

(22) Date of filing: **03.06.2020**

(51) International Patent Classification (IPC):
**G16C 20/30** *(2019.01)*

(52) Cooperative Patent Classification (CPC):
**G16C 20/30**

(86) International application number:
**PCT/EP2020/065263**

(87) International publication number:
**WO 2020/245140 (10.12.2020 Gazette 2020/50)**

(54) **METHOD FOR DETERMINING AT A CURRENT TIME POINT A PRESERVATION STATE OF ONE PRODUCT AND COMPUTER SYSTEM FOR CARRYING OUT SAID METHOD**

VERFAHREN ZUM BESTIMMEN EINES KONSERVIERUNGSZUSTANDES EINES PRODUKTS ZU EINEM AKTUELLEN ZEITPUNKT UND COMPUTERSYSTEM ZUR DURCHFÜHRUNG DES VERFAHRENS

PROCÉDÉ POUR DÉTERMINER AU NIVEAU D'UN POINT TEMPOREL ACTUEL UN ÉTAT DE CONSERVATION D'UN PRODUIT ET SYSTÈME INFORMATIQUE POUR EFFECTUER LEDIT PROCÉDÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.06.2019 EP 19305709**

(43) Date of publication of application:
**06.04.2022 Bulletin 2022/14**

(73) Proprietor: **Sanofi Pasteur**
**69007 Lyon (FR)**

(72) Inventor: **BRASS, Olivier**
**75008 Paris (FR)**

(74) Representative: **Santarelli**
**Tour Trinity**
**1 bis Esplanade de la Défense**
**92035 Paris La Défense Cedex (FR)**

(56) References cited:
**KR-A- 20060 087 098      US-A1- 2004 212 509**

- **Https://www.youtube.com/watch? v=e2kPz0DgpXg published on 02/05/2018. This was cited by a third party and is considered the closest prior art. For other more distant documents cited by the third party, see the document list in the file wrapper filed on 03-12-2021.**
- **CLANCY DON ET AL: "Kinetic Model Development for Accelerated Stability Studies", AAPS PHARMSCITECH, SPRINGER US, NEW YORK, vol. 18, no. 4, 15 July 2016 (2016-07-15), pages 1158 - 1176, XP036223790, DOI: 10.1208/ S12249-016-0565-4**
- **NAVERSNIK KLEMEN ET AL: "Humidity- corrected Arrhenius equation: The reference condition approach", INTERNATIONAL JOURNAL OF PHARMACEUTICS, ELSEVIER, NL, vol. 500, no. 1, 21 January 2016 (2016-01-21), pages 360 - 365, XP029416981, ISSN: 0378-5173, DOI: 10.1016/J.IJPHARM.2016.01.047**

- QUINTAS ET AL: "Modelling autocatalytic behaviour of a food model system-Sucrose thermal degradation at high concentrations", JOURNAL OF FOOD ENGINEERING, BARKING, ESSEX, GB, vol. 78, no. 2, 1 January 2007 (2007-01-01), pages 537 - 545, XP005584404, ISSN: 0260-8774, DOI: 10.1016/J.JFOODENG.2005.10.031

- EGAN W ET AL: "Basic principles of stability", BIOLOGICALS, ACADEMIC PRESS LTD., LONDON, GB, vol. 37, no. 6, 1 November 2009 (2009-11-01), pages 379 - 386, XP026748615, ISSN: 1045-1056, [retrieved on 20090831], DOI: 10.1016/J.BIOLOGICALS.2009.08.012

**Description**

FIELD OF THE INVENTION

The invention generally relates to groups of products made in a batch according to a predefined specification.

The invention relates more specifically to such groups in which the products have a quantitative attribute taking a value representative of the preservation state of the product, the quantitative attribute value evolving as a function of time and temperature, the product having a preservation state (for instance meeting or not meeting acceptance criteria) depending on the quantitative attribute value.

BACKGROUND ART

It is known that pharmaceutical products for human use are examples of such products and that harmonization works were performed on the conduct of stability studies for pharmaceutical products.

In particular, the International Council for Harmonisation of Technical Requirements for Pharmaceuticals for Human Use (ICH) provides guidelines on such stability studies, known as Q1A - Q1F amongst which is Q1A(R2) entitled "Stability Testing Of New Drug Substances And Products" and Q1E entitled "Evaluation for Stability Data".

The purpose of a ICH stability study is to establish, based on testing a minimum of three batches of the drug substance or product, a retest period (drug substance) or shelf life (drug product) and label storage instructions applicable to all future batches manufactured and packaged under similar circumstances.

Q1A(R2) guideline details principles for generating stability data and Q1E describes when and how extrapolation can be considered when proposing a retest period (drug substance) or a shelf life (drug product) that extends beyond the period covered by the available data generated from a stability study under the so-called long-term storage condition, such as twelve months in a refrigerator at 5°C $\pm$ 3°C.

The stability data are generated for the critical quality attributes likely to influence the quality and performance of the drug substance or product.

Q1E guideline explains that in general, certain quantitative chemical attributes (e.g., assay, degradation products, preservative content) for a drug substance or product can be assumed to follow zero-order kinetics during long-term storage.

Q1E guideline further explains that data for these attributes are therefore amenable to the type of statistical analysis described in its Appendix B, including linear regression and poolability testing; that although the kinetics of other quantitative attributes (e.g., pH, dissolution) is generally not known, the same statistical analysis can be applied, if appropriate; and that qualitative attributes and microbiological attributes are not amenable to this kind of statistical analysis.

Q1E guideline thus discloses a way of extrapolating a law of evolution - as a function of time - of a quantitative attribute value. With the extrapolated law of evolution, it is possible to determine the quantitative attribute value at time points different from the experimental time points at which the stability data were generated, in particular time points beyond the period covered by the stability data. The preservation state of the product (for instance within or out of acceptance criteria) can thus be determined at a current time point different from the experimental time points.

Such extrapolation is very useful for knowing the preservation state of one product in predefined conditions of storage, for instance the so-called long-term storage condition, such as twelve months in a refrigerator at 5°C $\pm$ 3°C.

More recently, the World Health Organization (WHO) Guidelines on Stability Evaluation Of Vaccines states that statistical modelling such as regression analysis may be used to analyse data from stability studies and states that modelling can be performed after three or more stability time points have been obtained.

https: //www.youtube.com/watch?v=e2kPz0DgpXg published on 02/05/2018, describes the "AKTS®-Thermokinetics® software - Prediction of Shelf Life of Materials by Kinetic Analysis", that provides prediction of the shelf life of materials from forced degradation studies based on different analytical techniques by using advanced kinetic and statistical analyses. This described "Thermal aging version" provides kinetic analysis, evaluation of kinetic models and parameters, screening of kinetic models, ranking of kinetic models according to statistical criteria AIC & BIC, prediction of the reaction course using chosen kinetic model and determination of prediction bands (95% confidence).

SUMMARY OF THE INVENTION

The invention is directed to an improved method for determining the preservation state of this kind of products, such method having good precision performance even with a limited amount of experimental stability data.

The invention provides accordingly a method for determining at a current time point a preservation state of one product of a group of products made in a batch according to a predefined specification, each product of said group of products having a quantitative attribute taking a value evolving as a function of time and temperature, said preservation state of said one product depending on said quantitative attribute value of said one product at said current time point, said method including:

(i) the step of determining a law of evolution - as a function of time - of said quantitative attribute value, including the step of receiving an experimental stability data set by conducting experiments in which a plurality of reference products for said group of products is stored at a constant temperature within predefined tolerances and in which at a plurality of different experimental time points - including an experimental initial time point - respective quantitative attribute values of respective reference products are determined and recorded; and including the step of extrapolating said law of evolution from said experimental data set;

(ii) the step of determining at said current time point the quantitative attribute value given by the law of evolution determined at step (i), whereby said current time point can be different from said experimental time points; and

(iii) the step of determining said preservation state of said one product at said current time point using the quantitative attribute value determined at step (ii);

wherein:

said method further includes the step of providing a computer system in which is stored a plurality of models of evolution of said quantitative attribute value as a function of time and temperature, each said model having between three and nine parameters, said parameters including a quantitative attribute value at an initial time point, hereinafter termed initial quantitative attribute value parameter, said computer system including an interface for entering said experimental stability data set, including an equation resolution tool for computing a previously entered experimental stability data set for finding for each said model best fitting values for its parameters, and including an estimator production tool for finding predetermined estimators for each model, said estimators including a physico-chemical parameter likelihood estimator, a Bayesian Information Criterion, a Fischer test, a t-test and a fit distance, said physico-chemical parameter likelihood estimator being based at least on a comparison between initial quantitative attribute values in the experimental data set and the best fitting value found for said each model for the initial quantitative attribute value parameter, said fit distance being based on a comparison between the values in the experimental data set and the corresponding values given by said each model;

in said step of producing an experimental stability data set, said plurality of reference products is stored at least at two different predetermined constant temperatures within predefined tolerances;

- said step of extrapolating said law of evolution from said experimental stability data set includes the following steps:

a) the step of receiving said experimental stability data set into said computer system through said interface;

b) the step of computing said experimental stability data set with said equation resolution tool in said computer system and the step of recording the found best fitting values for the parameters of each said model;

c) the step of further computing said experimental stability data set with said estimator production tool in said computer system and the step of recording the found estimators for each model; and

d) the step of selecting a best model amongst said models based on the respective found estimators, whereby the determined law of evolution of the quantitative attribute value is the selected best model with the found best fitting values for its parameters;

wherein:

step (ii) is carried out by calculation using successive temperature values taken by said one product as from an initial time point up to said current time point by entering into the computer system through the interface the time and a temperature profile over the time, the quantitative value calculation tool determining the quantitative attribute value at the current time point by successive iterations, that is first determining the quantitative attribute value after a first temperature step of the temperature profile at the beginning of the temperature profile, in which the temperature is constant, starting with the quantitative attribute value at the beginning of the temperature profile, then iteratively determining the quantitative attribute value after each other temperature step of the temperature profile in which the temperature is constant, starting with the quantitative attribute value at the end of the previous temperature step, until the current time point,

step (i) further includes a determination of a confidence interval for the quantitative attribute values calculated with the determined law of evolution,

step (iii) includes the step of determining if the confidence interval for the value calculated at the current time point includes a predetermined rejection threshold value; and

the step of selecting a best model selects a model that enables to find significant values for the confidence intervals of the parameters, and if at least two models enable to find significant values for the confidence intervals of the parameters, the step of selecting a best model selects the model meeting all the following criteria:

- the model for which the aggregated likelihood estimator is positive, the aggregated likelihood estimator being positive except

if at least one of the physico-chemical likelihood estimator, the Fisher test and the t-test is negative, if the difference between the found initial quantitative attribute value parameter and the initial experimental quantitative attribute value is greater than a predetermined threshold or if the found initial quantitative attribute value parameter is negative,

- the model having a found initial quantitative attribute value parameter closest to the quantitative attribute value of the product at the initial time point,
- the model having the lowest fit distance, and
- the model having the lowest Bayesian Information Criterion if the number of parameters is the same for two models.

The method according to the invention enables determining the preservation state of the product also in conditions of storage in which the temperature did not remain constant within predefined tolerances, for instance with a period of storage in a refrigerator followed by a period of travel at ambient temperature followed by a period of storage in a refrigerator.

Indeed, in the method according to the invention, the determined law of evolution is a function of time and temperature (and not of time only) and the successive temperatures taken by the product as from the initial time are used for determining the quantitative attribute value at the current time point.

Unlike commercially available software, even based on Arrhenius law (such as software commercialized by companies called AKTS and ASAP), the invention enables to obtain good precision performance even with a limited amount of experimental stability data.

Indeed, the invention is based on the observation that having - as in commercially available software - a single model involving an important number of parameters while providing a single statistical score for evaluating the quality of the found law of evolution, is not appropriate here because the amount of the experimental data set is low; and that in fact it is better to have a plurality of phenomenological models and to provide a plurality of estimators for discriminating the most appropriate model.

It is understood that in the present memorandum a parameter is a constant value initially unknown making part of a relation which becomes - further to the determination of the value of each parameter on the basis of an experimental data set - a law of evolution giving the quantitative attribute value as a function of time and temperature.

It is also understood that in the present memorandum a model is different from another model if they are expressed with different relations.

For instance, if a first relation is $1/(1/C0^2 + 2kt)^{1/2}$ and and a second relation is $1/(1/C0^{n-1} + k(n-1)t)^{1/(n-1)}$ with $k = A \exp(-Ea/RT)$, with C which is the quantitative attribute value, t which is the time, T which is the temperature, C0 which is a first parameter, A which is a second parameter, Ea which is a third parameter and n which is a fourth parameter, the first relation is the expression of a first model with three parameters and the second relation is the expression of a second model with four parameters, different from the first model.

It is noted that if the fourth parameter n is equal to 3 and the first to third parameters have the same values in the first model and the second model, the law of evolution given by the first model and the second model is the same, but this does mean that the second model is the same as the first model.

According to advantageous features:

- said parameters further include at least one of a parameter representative of an activation energy Ea and a parameter representative of a pre-exponential factor A and said physico-chemical likelihood parameter estimator is negative if Ea is lesser than 50 kJ/mol or greater than 1000 kJ/mol or if A is lesser than 1 kJ/mol or greater than 300 kJ/mol;
- said parameters further include at least one parameter representative of an order of reaction and said physico-chemical likelihood parameter estimator is negative if said at least one parameter representative of an order of reaction is lesser than 0 or greater than 10;
- said parameters further include a parameter $\alpha$ representative of a proportion of subpopulation or representative of a fraction value at an initial time and said physico-chemical likelihood estimator is negative if $\alpha$ is lesser than 0 or greater than 1;

- said estimators further include a quantitative statistics estimator based on at least one of a Fisher test and a t-test;
- said step of providing a computer system includes selecting said computer system as having a user interface displaying an aggregated likelihood estimator which is negative if said physico-chemical likelihood estimator is negative or said quantitative statistics estimator is negative;
- said step of providing a computer system includes selecting said computer system as having a user interface displaying visual indications representative of said estimators found by said estimator production tool and wherein said step of selecting a best model amongst said models is carried out by a user taking into account said visual indications representative of said estimators found by said estimator production tool;
- each said model has between three and eight parameters;
- the method further includes the step of determining a modified experimental stability data set, the step of providing the modified experimental data set and the step of carrying out again step (i) to step (iii) with said modified experimental stability data set;
- said current time point is the present moment;
- said steps (ii) and (iii) are carried out by an electronic time temperature indicator (eTTI); and/or
- said current time point is a future moment.

The invention is also directed, in a second aspect, to a computer system for carrying out the method as disclosed above.

BRIEF DESCRIPTION OF THE DRAWINGS

The description of the invention continues now with a detailed description of example embodiments given hereinafter by way of non-limiting illustration and with reference to the appended drawings. In the drawings:

- Figure 1 illustrates schematically a group of products made in a batch according to a predefined specification;
- Figure 2 illustrates a known method for enabling to determine the preservation state of one product at a current time point;
- Figure 3 illustrates schematically a computer system involved in the method according to the invention;
- Figure 4 is a graph of an experimental data set produced for determining a law of evolution of a quantitative attribute value; and
- Figure 5 is a graph similar to Figure 4 but showing also the determined law of evolution of a quantitative attribute value.

DETAILED DESCRIPTION OF EXAMPLE EMBODIMENTS

Background - disclosed but not claimed

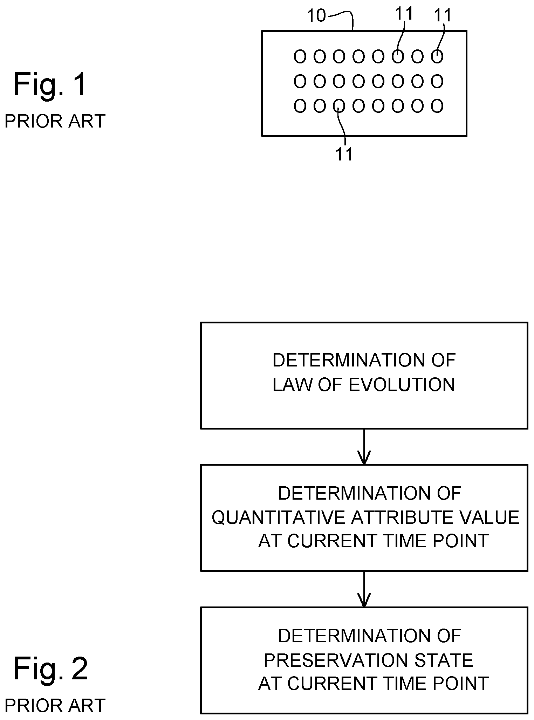

Figure 1 illustrates a group 10 of products 11 made in a batch according to a predefined specification, so that the products 11 are identical.

The products 11 are perishable and there is thus a need to determine at a current time point, such as the present time point or a time point in the future, if a product 11 still has a good preservation state.

The preservation state of a product 11 at a current time point depends on the value taken by a quantitative attribute of the product 11 evolving as a function of time and temperature.

Here, the product 11 is a vaccine dose to be administered to a patient and the quantitative attribute is the concentration of an active component in the vaccine dose.

Indeed, some active components of the vaccine dose are subject to a degradation reaction so that their quantity in the vaccine dose decreases over time and may fall below a specified efficacy threshold under which the therapeutic efficacy of the vaccine is considered lost.

The preservation state of the product 11 is considered good if the concentration of active component is over the threshold and considered bad if the concentration of active component is below the threshold.

For enabling to determine the preservation state of one product 11 at a current time point, a known method is illustrated on Figure 2.

This method is disclosed in the guidelines provided by the International Council for Harmonisation of Technical Requirements for Pharmaceuticals for Human Use (ICH) known as Q1A - Q1F amongst which is Q1A(R2) entitled "Stability Testing Of New Drug Substances And Products" and Q1E entitled "Evaluation for Stability Data".

This known method includes:

(i) the step of determining the law of evolution - as a function of time - of the quantitative attribute value such as the concentration of an active component in a vaccine dose;

(ii) the step of determining at the current time point the quantitative attribute value given by the law of evolution determined at step (i); and

(iii) the step of determining the preservation state of one product 11 at the current time point using the quantitative attribute value determined at step (ii).

For carrying out step (i), experiments are conducted on the stability of the products 11.

In these experiments, certain products 11 randomly selected in the group 10 are stored at different constant temperatures within predefined tolerances. At a plurality of different experimental time points - including an experimental initial time point - the quantitative attribute value of the respective products under experiment are determined and recorded.

An experimental data set is thus produced, each data being a quantitative attribute value at time t for a product 11 stored at a temperature T.

Then, step (i) is finalised by carrying out the step of extrapolating the law of evolution from the experimental data set, assuming that the quantitative attribute value follows zero-order kinetics during long-term storage and that the quantitative attribute value is amenable to certain type of statistical analysis described in Appendix B of Q1E ICH guideline, including linear regression and poolability testing.

With the extrapolated law of evolution, carrying out step (ii) enables to determine the quantitative attribute value at time points different from the experimental time points at which the experimental stability data were generated, in particular time points beyond the period covered by the experimental stability data.

From the quantitative attribute value determined at step (ii), step (iii) gives the preservation state of the product, for instance within or out of acceptance criteria involving a threshold for the quantitative attribute value.

In practice, the law of evolution determined on the basis of one group of products such as the group 10 is used for other groups of products made in a batch according to the same predefined specification, the samples (product 11 on which the experiments were conducted) taken from group 10 being considered as reference products for the other group of products.

In practice, it is often checked that the determined law of evolution remains the same when it is determined from experimental stability data set generated from samples taken in other groups of products made in a batch according to the same predefined specification, for instance the determined law of evolution is determined with three successive groups of products made in a batch according to the same predefined specification.

In the method according to the invention, the law of evolution is also extrapolated from an experimental data set but on basis much more advanced than merely assuming that the quantitative attribute value follows zero-order kinetics during long-term storage and that the quantitative attribute value is amenable to certain type of statistical analysis described in Appendix B of Q1E ICH guideline, including linear regression and poolability testing.

Computer system involved in the method according to the invention

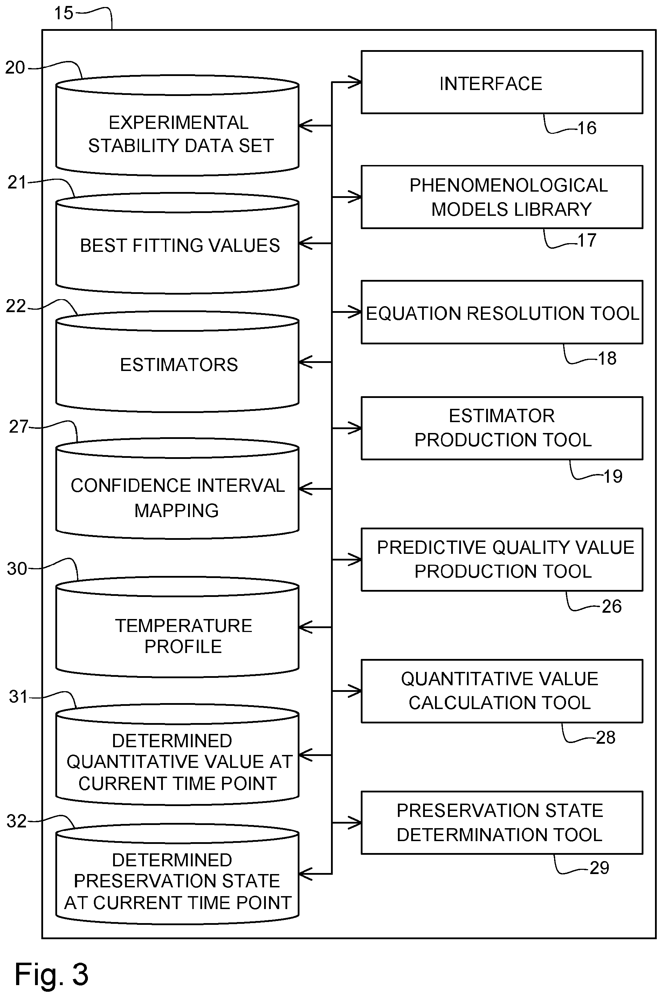

Figure 3 illustrates schematically a computer system 15 for helping to extrapolate from an experimental data set as described above a law of evolution of the quantitative attribute value as a function of time and temperature.

The computer system 15 includes an interface 16 for input and output of data, a phenomenological models library 17, an equation resolution tool 18, an estimator production tool 19, a storage 20 for the experimental stability data set, a storage 21 for best fitting values of parameters found by the equation resolution tool 18 and a storage 22 for estimators found by the estimator production tool 19.

Here, the computer system 15 further includes a predictive quality value production tool 26 and a storage 27 for a confidence interval mapping produced by the predictive quality value production tool 26.

Here, further to helping to extrapolate the law of evolution of the quantitative attribute value as a function of time and temperature, the computer system 15 is also able to determine at a current time point the quantitative attribute value using the previously determined law of evolution and to determine the preservation state of one product 11 at this current time point using the previously determined quantitative attribute value at this current time point.

The computer system 15 further includes accordingly a quantitative value calculation tool 28, a preservation state determination tool 29, a storage 30 for a temperature profile, a storage 31 for a determined quantitative value at a current time point and a storage 32 for a determined preservation state at a current time point.

It is noted that a distinct computer system such as an electronic Time Temperature Indicator (eTTI) can be configured with a similar quantitative value calculation tool, a similar preservation state determination tool, a similar storage for a temperature profile, a similar storage for a determined quantitative value at a current time point and a similar storage for a determined preservation state at a current time point, with of course an interface for input and output of data.

The computer system 15 is carried out here with a stand-alone computer such as a PC or Mac running a suitable program (for instance based on Matlab®), the interface 16 being the local display, keyboard and ports of the stand-alone computer. Alternatively, the computer system is carried out differently, for instance with a server running a suitable program (for instance based on Matlab®), the interface 16 being carried out with a client, a communication link and the local interface of the server for connecting the client through the communication link.

<u>Library 17</u>

In the library 17 are stored a plurality of phenomenological models of evolution of the quantitative attribute value of a product 11, here the concentration of active component in a vaccine dose.

The phenomenological models are each based on the Arrhenius relationship between the kinetic constant k of a reaction and the temperature T. The Arrhenius relationship is based on the collision theory explaining that, for a reaction to occur between two particles or more, it is necessary that a collision occurs between these particles, in the right direction and with enough energy.

The Arrhenius relationship is:

$$k = A \exp(-Ea/RT)$$

the kinetic constant k being the number of reactions per unit of time; the pre-exponential factor A being the total number of collisions per unit of time; and the exponential factor $\exp(-Ea/RT)$ being the probability that a collision will result in a reaction, Ea being the energy of activation of the reaction, T the temperature in °K and R the perfect gas constant.

<u>Models called Order 0, Order 1, Order 2, Order 3 and Order n</u>

For certain models in the library 17, called Order 0, Order 1, Order 2, Order 3 and Order n, it is assumed that the reaction degradation of the quantitative attribute value of a product 11, here the concentration of active component in a vaccine dose, follows the below law of chemical kinetics:

$$dC/dt = k\, f(C)$$

C being the quantitative attribute value (here the concentration of active component in a vaccine dose), t being the time and f being the respective function on which is based the model called Order 0, Order 1, Order 2, Order 3 or Order n.

Table 1 below gives for each of these five models the expression of f(C), the expression of dC/dt and the expression of C(t) which is the analytic solution of integrating dC/dt at constant temperature.

Table 1

| Name of model | Expression of f(C) | Expression of dC/dt | Expression of C(t) |
|---|---|---|---|
| Order 0 | $-1$ | $-k$ | $C_0 - k\, t$ |
| Order 1 | $-C$ | $-k\, C$ | $C_0 \exp(-k\, t)$ |
| Order 2 | $-C^2$ | $-k\, C^2$ | $1/(1/C_0 + k\, t)$ |
| Order 3 | $-C^3$ | $-k\, C^3$ | $1/(1/C_0^2 + 2k\, t)^{1/2}$ |
| Order n | $-C^n$ | $-k\, C^n$ | $1/(1/C_0^{n-1} + k\,(n-1)\, t)^{1/(n-1)}$ |

In the expression of C(t), $C_0$ is the quantitative attribute value (concentration of active component in a vaccine dose) at the initial time point.

It results from the expression of C(t) and from the Arrhenius relationship giving k as a function of the pre-exponential factor A, of the activation energy Ea and of the temperature T, that the models called Order 0, Order 1, Order 2 and Order 3 have three parameters, namely A, Ea and $C_0$; and that the model called order n has four parameters, namely n further to the parameters of the models called Order 0, Order 1, Order 2 and Order 3 (A, Ea and $C_0$).

The value of the temperature T at which the product is stored and the values of parameters A, Ea and $C_0$ (and also n for the model called Order n) enable to determine the quantitative attribute value C at a time t.

It is also possible to determine the quantitative attribute value C at a time t if the temperature did not remain constant, provided that the temperature profile over the time is known (for example 60 days at 5°C as from the initial time, then 1 day at 25°C and then 10 days at 5°C) and provided that the determination is made iteratively, that is successively for each temperature step (for instance in the above example first determining the quantitative attribute value at 60 days starting with C0, then determining the quantitative attribute value at 61 days starting with the quantitative attribute value at 60 days for a duration of 1 day, then determining the quantitative attribute value at 71 days starting with the quantitative attribute value at 61 days for a duration of 10 days).

The model called Order 0 corresponds to a reaction of which the kinetics is independent of the concentrations of the components of the couple reactant/substrate. In biology, this is the case for certain enzyme-catalyzed reactions. In practice, this model applies as long as the substrate concentration remains high, for instance at the beginning of the reaction.

The model called Order 1 corresponds to a reaction of which the kinetics depends on the concentration of only one of the components of the couple reactant/substrate. This is the case for certain reactions of the same type as hydrolysis in aqueous media, oxidation at the air/water interface or enzyme catalysis.

The model called Order 2 corresponds to a reaction of which the kinetics depends on the concentrations of both of the components of the couple reactant/substrate if the reaction is of the same type as polysaccharide saponification or as protein deamidation, or depends on the concentration of only one of the components of the couple reactant/substrate if the reaction is of the same type as protein dimerization.

The model called Order 3 corresponds to a reaction of which the kinetics depends on the concentration of both of the components of the couple reactant/substrate if one of them is bimolecular and if the reaction is of the same type as protein trimerisation.

The model called Order n corresponds to a reaction of which the kinetics depends on the concentration of both of the components of the couple reactant/substrate if one or both of them is/are bimolecular or multimolecular and if the reaction is of the same type as an $\alpha$-helix protein structure break or an oligomerization greater than 3.

It is noted that the model called Order n gives the same law of evolution as the model called Order 0, Order 1, Order 2 and Order 3 if n is respectively equal to zero, one, two and three. In practice, the model called Order n is useful for n which is not an integer number and/or is greater than three.

Model called Parallel reaction

Another model in the library 17 is called Parallel reaction. It is assumed the same as for the model called Order n except that two reactions (and not a single one) are considered as simultaneously going on, respectively reaction 1 involving parameters A1, Ea1, C01 and n1 for a partial quantitative attribute C1 and reaction 2 involving parameters A2, Ea2, C02 and n2 for a partial quantitative attribute C2, the quantitative attribute value C being the sum of the partial quantitative attributes C1 and C2 (in other words C = C1 + C2) with C1 = $\alpha$ C and C2 = (1 - $\alpha$) C, $\alpha$ being called the proportion of subpopulation.

There are thus eight parameters for the model called Parallel reaction, namely C0, $\alpha$, A1, Ea1, n1, A2, Ea2 and n2.

The value of the temperature T at which the product is stored and the values of parameters C0, $\alpha$, A1, Ea1, n1, A2, Ea2 and n2 enable to determine the quantitative attribute value C at a time t, by numerical integration.

It is also possible to determine the quantitative attribute value C at a time t if the temperature did not remain constant, provided that the temperature profile over the time is known and provided that the determination is made iteratively as explained above.

Model called Finke-Watzky

Another model in the library 17 is called Finke-Watzky. Further to the quantitative attribute value C (here the concentration of active component in a vaccine dose) a further quantitative attribute value N is considered (here the concentration of degraded active component in a vaccine dose), with the following relationships between N and C:

$$dN/dt = k1\ C$$

$$dC/dt = -k1\ C - k2\ C\ N$$

k1 and k2 being respective kinetic constants of two different Arrhenius relationship, namely:

$$k1 = A1\ \exp(-Ea1/RT)$$

$$k2 = A2\ \exp(-Ea2/RT)$$

There are thus five parameters for the model called Finke-Watzky, namely C0, A1, Ea1, A2 and Ea2.

The value of the temperature T at which the product is stored and the values of parameters C0, A1, Ea1, A2 and Ea2 enable to determine the quantitative attribute value C at a time t, by numerical integration.

It is also possible to determine the quantitative attribute value C at a time t if the temperature did not remain constant, provided that the temperature profile over the time is known and provided that the determination is made iteratively as explained above.

The model called Finke-Watzky corresponds to a reaction of aggregation, like nucleation or germination, which is common in media with proteins, in particular if the latter are concentrated.

Model called Prout-Tompkins

Another model in the library 17 is called Prout-Tompkins. This model is also known as Sestak n/m. It is assumed that the reaction degradation of the quantitative attribute value (here the concentration of active component in a vaccine dose) is at a fraction $\xi$ at a time t and that the fraction $\xi$ follows the below law of chemical kinetics:

$$d\xi/dt = -k\,\xi^n\,(1-\xi)^m$$

with $\xi = 1$ when the reaction is finished and $\xi$ greater than 0 (and lesser than 1) at the start of the reaction.

For simplifying the notations, the value of $\xi$ at the initial time is noted $\alpha$ in the following. It is noted that this $\alpha$ has not the same phenomenological significance as $\alpha$ in the model called Parallel reaction.

There are thus six parameters for the model called Prout-Tompkins, namely C0, A, Ea, n, m and $\alpha$.

The value of the temperature T at which the product is stored and the values of parameters C0, A, Ea, n, m and $\alpha$ enable to determine the quantitative attribute value C at a time t, by numerical integration.

It is also possible to determine the quantitative attribute value C at a time t if the temperature did not remain constant, provided that the temperature profile over the time is known and provided that the determination is made iteratively as explained above.

The model called Prout-Tompkins corresponds to a reaction that is autocatalytic or progressively accelerated.

Model called Sestak n/p.

Another model in the library 17 is called Sestak n/p. It is assumed the same as for the model called Prout-Tompkins except that the law of chemical kinetics followed by the fraction $\xi$ is:

$$d\xi/dt = -k\,\xi^n\,\log(1-\xi)^p$$

There are thus six parameters for the model called Sestak n/p, namely C0, A, Ea, n, p and $\alpha$.

The value of the temperature T at which the product is stored and the values of parameters C0, A, Ea, n, p and $\alpha$ enable to determine the quantitative attribute value C at a time t, by numerical integration.

It is also possible to determine the quantitative attribute value C at a time t if the temperature did not remain constant, provided that the temperature profile over the time is known and provided that the determination is made iteratively as explained above.

The model called Sestak n/p corresponds to a reaction of order n that is progressively accelerated with a factor p limiting the diffusion of the reactants or of the degradation products. This reaction is of the same type as a hydrolysis in solid formulation.

Model called Sestak m/p

Another model in the library 17 is called Sestak m/p. It is assumed the same as for the model called Prout-Tompkins except that the law of chemical kinetics followed by the fraction $\xi$ is:

$$d\xi/dt = k\,(1-\xi)^m\,\log(1-\xi)^p$$

There are thus six parameters for the model called Sestak m/p, namely C0, A, Ea, m, p and $\alpha$.

The value of the temperature T at which the product is stored and the values of parameters C0, A, Ea, m, p and $\alpha$ enable to determine the quantitative attribute value C at a time t, by numerical integration.

It is also possible to determine the quantitative attribute value C at a time t if the temperature did not remain constant, provided that the temperature profile over the time is known and provided that the determination is made iteratively as explained above.

The model called Sestak m/p corresponds to a reaction of order m that is autocatalytic with a factor p limiting the reaction product or the diffusion of the reactant or of the degradation products. This reaction is of the same type as an oligomerization in solid formulation.

Model parameters recapitulation

Table 2 below recapitulates the parameters in the ten phenomenological models in the library 17:

Table 2

| Name of model | Model parameters | | | | | | | |
|---|---|---|---|---|---|---|---|---|
| Order 0 | A | Ea | C0 | | | | | |
| Order 1 | A | Ea | C0 | | | | | |
| Order 2 | A | Ea | C0 | | | | | |
| Order 3 | A | Ea | C0 | | | | | |
| Order n | A | Ea | C0 | n | | | | |
| Finke-Watzky | A1 | Ea1 | C0 | A2 | Ea2 | | | |
| Prout-Tompkins | A | Ea | C0 | n | m | $\alpha$ | | |
| Sestak n/p | A | Ea | C0 | n | p | $\alpha$ | | |
| Sestak m/p | A | Ea | C0 | m | p | $\alpha$ | | |
| Parallel reaction | A1 | Ea1 | C0 | A2 | Ea2 | $\alpha$ | n1 | n2 |

The phenomenological models in the library 17 thus have between three and height parameters.

Equation resolution tool 18

In the computer system 15, the equation resolution tool 18 is able to compute a previously entered stability data set present in the storage 20 so as to find for each model in the library 17 best fitting values for the parameters.

The best fitting values for the parameters are found by optimizing a function of the parameters selected as representative of the fit of C(t) to the experimental data set; the best fitting values of the parameters being the values for which the function is optimized.

Here, such function is the sum of the square of the differences between the values in the experimental data set and the corresponding values given by C(t); and the function is optimized by minimizing the sum of the square of the differences.

The optimization of the function is performed by running an iterative algorithm, here carried out with the fminsearch function of Matlab® which is based on the Nelder-Mead simplex algorithm, from a starting set of values for the parameters.

The values of the starting set are here randomly generated by the computer system 15, for instance by using a clock drift as a source of randomness.

The iterative algorithm is then run for each model from this starting set of values and the found best fitting values are recorded in the storage 21.

Estimator production tool 19

In the computer system 15, the estimator production tool 19 is able to find for each model in the library 17 various estimators for helping to evaluate the modelling quality of the model.

One estimator is directed to the physico-chemical likelihood of the found parameters. It is recalled that since the models are phenomenological, the parameters each have a physico-chemical meaning so that it is possible to assign to each parameter a range of likely values, the values out of the range being unlikely, for instance a negative value for the activation energy Ea.

Other estimators are based on quantitative statistics and qualitative (probabilistic) statistics.

Physico-chemical likelihood estimator of the found best fitting values for the parameters

This estimator can be positive (good likelihood) or negative (bad likelihood).

The physico-chemical likelihood estimator is positive except in the cases stated below for which the physico-chemical likelihood estimator is negative.

For the found initial quantitative attribute value parameter C0, a comparison is made with the initial experimental quantitative attribute value (initial quantitative attribute value in the experimental stability data set).

The physico-chemical likelihood estimator is negative if the difference between the found initial quantitative attribute value parameter C0 and the initial experimental quantitative attribute value is greater than a predetermined threshold. The physico-chemical likelihood estimator is also negative if the found initial quantitative attribute value parameter C0 is lesser than 0.

Here, if the analytical standard deviation of the experimental stability data set is unknown, the threshold is 30% of the initial experimental quantitative attribute value (or 0.3 if the values are given on a logarithmic scale).

If the analytical standard deviation of the experimental stability data set is known, then the threshold is the value of the analytical standard deviation.

The analytical standard deviation is conventionally a statistical measure of the precision of the measurements from which was obtained the experimental stability data set.

For the found parameter Ea in principle representative of an activation energy, the physico-chemical likelihood estimator is negative if Ea is lesser than 50 or greater than 1000 kJ/mol. Alternatively, the range 50-1000 kJ/mol is replaced by a narrower range, such as 50-500 kJ/mol or 50-300 kJ/mol.

For the found parameter A in principle representative of a pre-exponential factor, the physico-chemical likelihood estimator is negative if A is lesser than 1 or greater than 300 kJ/mol. Alternatively, the range 1-300 kJ/mol is replaced by a narrower range, such as 20-200 kJ/mol.

For the found parameter $\alpha$ in principle representative of the proportion of subpopulation (model called Parallel reaction) or representative of the fraction value at the initial time (models called Prout-Tompkins, Sestak n/p and Sestak m/p), the physico-chemical likelihood estimator is negative if $\alpha$ is lesser than 0 or greater than 1.

For the found parameters n, m and p in principle each representative of an order of reaction, the physico-chemical likelihood estimator is negative if n, m or p is lesser than 0 or greater than 10. Alternatively, the range 0-10 is replaced by a narrower range, such as 0-3.

Estimators based on qualitative statistics

One estimator based on qualitative statistics is a fit distance based on a comparison between the values in the experimental stability data set present in the storage 20 and the corresponding values given by the model.

The fit distance is here the square root of the sum of the squares of the differences between the values in the experimental stability data set and the corresponding values given by the model. The smaller the value of the fit distance, the better the model fits to the experimental data set.

Another estimator based on qualitative statistics is the Bayesian Information Criterion (BIC). As is well known, this criterion makes it possible to estimate the bias induced by the number of parameters on the modelling quality. The lower the value of the BIC, the lower the bias induced by the number of parameters.

The BIC is interesting when comparing two models having a different number of parameters: if the lowest value of the BIC is for the model having the highest number of parameters, the modelling quality is in principle better for the model having the highest number of parameters.

Other estimators based on qualitative statistics useful for comparing two models are the well known Akaike Information Criterion (AIC) and the likelihood ratio test (LLH). The AIC and LLH may be calculated using the respective functions available in Matlab®.

Estimators based on quantitative statistics

One estimator based on quantitative statistics is the result (positive or negative) of the Fisher test.

As is well known, the Fisher test is based on a comparison of the standard deviation of the values in the experimental stability data set (analytical standard deviation) with the standard deviation of the set of values given by the model at the corresponding time points (model standard deviation), so as to determine whether the analytical standard deviation is similar or lesser than the model standard deviation.

Another estimator based on qualitative statistics is the result (positive or negative) of the Student t-test.

The Student t-test is made only if the result of the Fisher test is positive.

As is well known, the Student t-test is based on a comparison of the mean of the values in the experimental stability data set (analytical mean) with the mean of the set of values given by the model at the corresponding time points (model mean). The Student t-test is here made using a 95% confidence level.

The estimators found by the estimator production tool 19 are recorded in storage 22.

Predictive quality value production tool 26

In the computer system 15, the predictive quality value production tool 26 is able to find for a selected model in the library 17 for which best fitting values are found a confidence interval mapping.

The confidence interval mapping includes a confidence interval (CI) for each of the found best fitting values. Such confidence intervals include each a lower value and an upper value between which is the best fitting value.

The lower and upper values are here found using a Bayesian method.

It should be noted that in certain cases, the experimental data set does not enable to find lower and upper values of the confidence interval which are significant, in particular for the models having the most numerous parameters.

The found confidence interval mapping or the absence of significant interval mapping is stored in the storage 27.

Quantitative value calculation tool 28

Based on the respective estimators found by the estimator production tool 19 and based on the initial quantitative attribute value parameter as compared with initial quantitative attribute values in the experimental stability data set, a best model amongst the models is selected.

Should the experimental data set does not enable to find significant confidence intervals for the parameters, it could be tried to modify the experimental data set such as by adding one or more experimental point(s) and/or decided to select another model for which the experimental data set enables to find significant confidence intervals.

The selected best model with the found best fitting values for its parameters thus becomes the determined law of evolution of the quantitative attribute value.

The quantitative value calculation tool 28 is accordingly configured through the interface 16.

The computer system 15 and in particular the quantitative value calculation tool 28 is thus aware of the determined law of evolution together with the confidence interval of each parameter value.

It thus becomes possible to ask to the quantitative value calculation tool 28 to determine the quantitative attribute value C at a current time point t by entering into the computer system 15 through the interface 16 the time t and the value of the constant temperature T at which the product is stored.

It is also possible to ask to the quantitative value calculation tool 28 to determine the quantitative attribute value C at a current time point t by entering into the computer system 15 through the interface 16 the time t and a temperature profile over the time (for example 60 days at 5°C as from the initial time, then 1 day at 25°C and then 10 days at 5°C), this profile being recorded in the storage 30.

The quantitative value calculation tool 28 determines the quantitative attribute value C at the current time point t by successive iterations, that is successively for each temperature step (for instance in the above example first determining the quantitative attribute value at 60 days starting with C0, then determining the quantitative attribute value at 61 days starting with the quantitative attribute value at 60 days for a duration of 1 day, then determining the quantitative attribute value at 71 days starting with the quantitative attribute value at 61 days for a duration of 10 days).

It is thus possible to determine the quantitative attribute value at time points different from the experimental time points at which the experimental stability data were generated, in particular time points beyond the period covered by the experimental stability data; and also to determine the quantitative attribute value with storage conditions in which the temperature did not remain constant.

The determined quantitative attribute value C at the current time point t is recorded in the storage 31.

Further to the quantitative attribute value C at the current time point t the quantitative value calculation tool is able, from the confidence intervals of the parameter values, to determine the confidence interval of the found quantitative attribute value C at the current time point. The determined confidence interval is recorded in the storage 31 together with determined quantitative attribute value C.

Preservation state determination tool 29

From the quantitative attribute value and associated confidence interval recorded in the storage 31, the preservation state determination tool 29 is able to determine the preservation state of the product, for instance within or out of acceptance criteria involving a threshold for the quantitative attribute value taking into account the associated confidence interval.

The determined preservation state of the product is recorded in the storage 32 and communicated to the user through the interface 16.

An example of carrying out the method according to the invention with the computer system 15 will now be described.

Example

In this example, certain products 11 randomly selected in the group 10 were stored at different constant temperatures within predefined tolerances and at a plurality of different experimental time points - including an experimental initial time point - the concentration in active component of the respective products under experiment were determined and recorded.

The experimental data set listed in Table 3 below was produced from such experiments in which the products 11 in the group 10 are doses of a formulation of vaccine against flu.

Table 3

| Time (days) | Temperature (°C) | SRD ($\mu$g HA/ml) |
|---|---|---|
| 0 | 4 | 798 |
| 5 | 4 | 769 |
| 12 | 4 | 670 |
| 20 | 4 | 906 |
| 27 | 4 | 766 |
| 37 | 4 | 635 |
| 0 | 25 | 798 |
| 2 | 25 | 755 |
| 5 | 25 | 746 |
| 8 | 25 | 650 |
| 12 | 25 | 578 |
| 16 | 25 | 569 |
| 20 | 25 | 630 |
| 23 | 25 | 543 |
| 27 | 25 | 524 |
| 34 | 25 | 548 |
| 37 | 25 | 460 |
| 0 | 37 | 798 |
| 2 | 37 | 603 |
| 5 | 37 | 574 |
| 8 | 3 | 288 |
| 12 | 37 | 333 |
| 16 | 37 | 332 |
| 20 | 37 | 316 |
| 23 | 37 | 316 |
| 27 | 37 | 297 |
| 34 | 37 | 274 |
| 37 | 37 | 261 |
| 0 | 45 | 798 |
| 2 | 45 | 350 |
| 5 | 45 | 248 |

(continued)

| Time (days) | Temperature (°C) | SRD (µg HA/ml) |
|---|---|---|
| 8 | 45 | 167 |
| 12 | 45 | 119 |
| 16 | 45 | 96 |
| 20 | 45 | 70 |
| 23 | 45 | 78 |
| 27 | 45 | 59 |
| 34 | 45 | 53 |
| 37 | 45 | 45 |

It is seen that the experiments were conducted with four different constant temperatures, respectively 4°C, 25°C, 37°C and 45°C, with one product 11 for each temperature. The tolerance for each constant temperature was ± 3°C. For each of the four different temperatures, the experiments were conducted during 37 days as from the initial time point (time of release of the group 10).

The quantitative attribute in the experimental data set listed in Table 3 is the HA Flu potency, determined by the SRID assay.

For each sample (product 11 on which the experiments were conducted), the initial HA Flu potency (value at the initial time point) is the same, namely 798 µg/ml.

Due to a degradation reaction, the HA Flu potency decreases over time but it is noted that certain values in the experimental data set are over the initial HA Flu potency value or over preceding values (see for instance at 20 days for 4°C and for 25°C). This is due to incertitude of measurement.

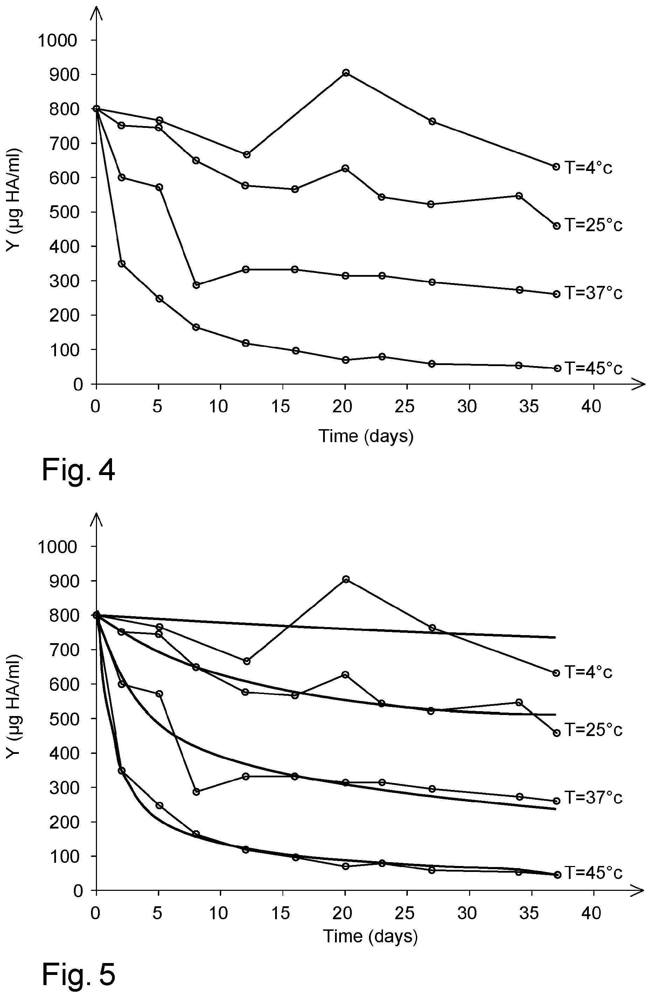

Figure 4 of the drawings is a graph of the experimental data set listed in Table 3 with the HA Flu potency (in µg/ml) on the vertical axis and the time (in days) on the horizontal axis.

The experimental data set listed in Table 3 above was entered into the computer system 15 and the equation resolution tool 18 was used for computing this experimental stability data set for finding for each model in the library 17 best fitting values for the parameters.

Best fitting values were found for each model in the library 17 and stored in the storage 21 of the computer system 15.

The found best fitting values and - when significant - the lower value and upper value of the confidence interval (CI) are listed in Tables 4 to 13 below, in which the values are in J/Mol:

Table 4

| Order 0 | A | Ea | C0 |
|---|---|---|---|
| Best fitting values | 52.886 | 129471.289 | 666.535 |
| Lower CI | 52.724 | 129146.852 | 666.080 |
| Upper CI | 53.046 | 129793.385 | 666.982 |

Table 5

| Order 1 | A | Ea | C0 |
|---|---|---|---|
| Best fitting values | 40.858 | 113117.477 | 726.194 |
| Lower CI | 40.690 | 112778.651 | 725.610 |
| Upper CI | 41.027 | 113457.974 | 726.777 |

Table 6

| Order 2 | A | Ea | C0 |
|---|---|---|---|
| Best fitting values | 40.834 | 128384.172 | 759.434 |
| Lower CI | 40.674 | 128060.431 | 758.822 |

(continued)

| Order 2 | A | Ea | C0 |
|---|---|---|---|
| Upper CI | 40.991 | 128702.354 | 760.047 |

Table 7

| Order 3 | A | Ea | C0 |
|---|---|---|---|
| Best fitting values | 43.999 | 152136.797 | 769.724 |
| Lower CI | 43.820 | 151771.009 | 769.096 |
| Upper CI | 44.179 | 152500.806 | 770.352 |

Table 8

| Order n | A | Ea | n | C0 |
|---|---|---|---|---|
| Best fitting values | 42.896 | 144912.841 | 2.719 | 768.109 |
| Lower CI | 42.594 | 144437.316 | 2.708 | 767.477 |
| Upper CI | 43.196 | 145391.814 | 2.732 | 768.746 |

Table 9

| Parallel reaction | A1 | Ea1 | A2 | Ea2 | n1 | n2 | C0 | $\alpha$ |
|---|---|---|---|---|---|---|---|---|
| Best fitting values | 32.47 | 85537.34 | 79.54 | 235742.74 | 0.93 | 2.36 | 796.3 | 0.34 |

Table 10

| Finke-Watzky | A1 | Ea1 | A2 | Ea2 | C0 |
|---|---|---|---|---|---|
| Best fitting values | 40.858 | 113117.477 | 43.228 | 217981.701 | 726.194 |

Table 11

| Prout-Tompkins | A | Ea | n | m | C0 | $\alpha$ |
|---|---|---|---|---|---|---|
| Best fitting values | 87.051 | 209820.663 | 5.880 | -0.443 | 2657.054 | 0.293 |

Table 12

| Sestak n/p | A | Ea | n | p | C0 | $\alpha$ |
|---|---|---|---|---|---|---|
| Best fitting values | 61.516 | 164608.740 | 2.140 | 1.188 | 779.613 | 1.000 |

Table 13

| Sestak m/p | A | Ea | m | p | C0 | $\alpha$ |
|---|---|---|---|---|---|---|
| Best fitting values | 73.219 | 188531.605 | -1.929 | 3.454 | 2116.135 | 0.369 |

No lower value and upper value of the confidence interval (CI) is listed in Tables 4 to 13 because the found values are too high for being significant.

In the computer system 15, the estimator production tool 19 was used for finding for each model the physico-chemical likelihood estimator, the fit distance, the BIC and the Fisher test and the t-test.

Table 14 below lists for each model the found distance, an aggregated likelihood estimator, the statistical distance, the BIC and a combined result of the Fisher test and the t-test. Also listed is the found initial quantitative attribute value parameter C0.

The combined result of the Fisher test and the t-test is positive if the result of the Fisher test is positive and the result of the t-test is positive. The combined result of the Fisher test and the t-test is negative if the result of the Fisher test is negative (it is recalled that here the t-test is made only if the result of the Fisher test is positive).

The aggregated likelihood estimator is positive except if at least one of the physico-chemical likelihood estimator and the combined result of the Fisher test and the t-test is negative.

Table 14

| Name of model | Aggregated likelihood estimator | C0 | Fit distance | BIC | Result of Fisher and t-test |
|---|---|---|---|---|---|
| Order 0 | Negative | 666.5354 | 677.0105 | 487.1686 | Negative |
| Order 1 | Negative | 726.1937 | 511.7331 | 465.3376 | Negative |
| Order 2 | Negative | 759.4342 | 382.5033 | 442.6345 | Negative |
| Order 3 | Negative | 769.7236 | 362.4647 | 438.4366 | Negative |
| Order n | Negative | 768.1091 | 360.0872 | 441.5876 | Negative |
| Parallel reaction | Positive | 796.3003 | 294.8143 | 440.6418 | Positive |
| Finke-Watzky | Negative | 726.1937 | 511.7331 | 472.6648 | Negative |
| Prout-Tompkins | Negative | 2657.0543 | 383.6597 | 453.8606 | Negative |
| Sestak n/p | Negative | 779.613 | 360.912 | 449.0931 | Negative |
| Sestak m/p | Negative | 2116.1353 | 368.0226 | 450.6149 | Negative |

Here, the computer system 15 displays Table 14 to the user so as to give him a basis for selecting one model amongst the ten models in the library 17.

One possibility for displaying the aggregated likelihood estimator is in the same column as the name of the models, with each cell giving the name of a respective model having a background taking one out of two predetermined colors, such as green for positive and orange for negative.

As mentioned above, further to the estimators generated by the estimator production tool 19, Table 14 lists also the found initial quantitative attribute value parameter C0.

It is indeed interesting for the user to make its own comparison between the value of the found initial quantitative attribute value parameter C0 and the experimental initial quantitative attribute value, for instance for taking into account experimental difficulties in measuring the experimental initial quantitative attribute value which are not reflected by the analytical standard deviation considered for generating the physico-chemical likelihood estimator of the found best fitting values for the parameters.

The user might thus keep a model despite a negative aggregated likelihood estimator.

In the present example, the aggregated likelihood estimator is positive only for Parallel reaction (it is negative for all the other models).

This is in favor of Parallel reaction.

As to the found initial quantitative attribute value parameter C0, it is recalled that in this example for each sample (product 11 on which the experiments were conducted), the initial HA Flu potency is 798 $\mu$g/ml.

The models having the found initial quantitative attribute value parameter C0 closest to 798 is Parallel reaction (796.3) followed by Sestak n/p (779.6), Order 3 (769.7) and Order n (768.1).

This is in favor of Parallel reaction.

As to the fit distance, the lower the fit distance the better the modelling quality.

The model having the lowest fit distance is Parallel reaction (294.81) followed by Order n (360.09), Sestak n/p (360.91) and Order 3 (362.46).

This is in favor of Parallel reaction.

As to BIC, the lower the BIC the better the modelling quality if the number of parameters is the same.

The model having the lowest BIC is Order 3 (438.43) followed by Parallel reaction (440.64), Order n (441.5876) and Order 2 (442.63).

It is recalled that the number of parameters is three for Order 3 and Order 2, is four for Order n and is eight for Parallel reaction.

This is in favor of Parallel reaction.

As to the Fisher test and t-test, only Parallel reaction is positive (it is negative for all the other models).

It thus appears that the best modelling quality is for Parallel reaction.

Figure 5 of the drawings is similar to Figure 4 but shows also the curves given by the model called Parallel reaction with the found best fitting values for the parameters.

It is seen that such modelling follows relatively closely the experimental data set.

However, as visible in table 9 above, the experimental data set listed in Table 3 does not enable to find significant values for the confidence intervals of the parameters of the model called Parallel reaction.

It was thus decided to select another model for which significant values were found, namely the model called Order n which is just behind the model called Parallel reaction as regards modelling quality.

With the selection of the model called Order n with the found best fitting values for the parameters, it becomes possible as explained above to ask to the quantitative value calculation tool 28 to determine the quantitative attribute value C at a current time point t by entering into the computer system 15 through the interface 16 the time t and the value of the constant temperature T at which the product is stored; and also to ask to the quantitative value calculation tool 28 to determine the quantitative attribute value C at a current time point t by entering into the computer system 15 through the interface 16 the time t and a temperature profile over the time recorded in the storage 30.

When the determined quantitative attribute value C at the current time point t is determined and recorded in the storage 31 together with the associated confidence interval, the preservation state determination tool 29 is able to determine the preservation state of the product, for instance within or out of acceptance criteria involving a threshold for the quantitative attribute value taking into account the associated confidence interval.

The determined preservation state of the product is recorded in the storage 32 and communicated to the user through the interface 16.

For finding a better law of evolution of the quantitative attribute value, it is possible to determine a modified experimental stability data set, to provide the modified experimental data set and to carry out again the method with the modified experimental stability data set.

For instance, the modified experimental stability data set is:

- the original experimental data set plus further experimental data from further experiments conducted in the meantime;
- the original experimental data set minus experimental data excluded as non-significant, for instance because one of the predetermined constant temperature is too high;
- a mix of the above, namely the original experimental data set from which certain experimental data are excluded while further experimental data are added; or
- a totally new experimental data set, with no common part with the original data set, such as from experiments on samples of a different group of products made in a different batch.

The modified experimental data set is for instance determined:

- with a view to provide a better set of predetermined estimators and thus ease the step of selecting a best model amongst the models;
- with a view to provide more precise values for the parameters of the selected best model and thus a more precise determination of the preservation state of said one product at the current time point, i.e. with a reduced confidence interval of the parameters; or
- with a view to check whether the law of evolution determined on the basis of the original experimental data set (from selected samples of the group of products) also apply to the totally new experimental data set (from selected samples of the different group of products).

In variants to the embodiments disclosed above, the product is different from a vaccine dose to be administered to a patient, for instance another pharmaceutical product, a cosmetic product, a synthetic chemistry product or a food product, such products different from a vaccine dose having a degradation following the Arrhenius law, with in particular the direction of variation of the quantitative attribute value remaining the same (always decrease or always growth), unlike microbiological degradation for which there is a change in the direction of variation.

In variants to the embodiments disclosed above, the phenomenological models of evolution of the quantitative attribute value as a function of time and temperature are different, for instance with up to nine parameters.

In variants to the embodiments disclosed above, the computer system includes also models of evolution of the quantitative attribute value as a function of time and temperature which are phenomenological and include more than nine parameters, for example ten parameters; and/or models that are not phenomenological; and/or models of evolution of the quantitative attribute value as a function of time and temperature and other factors such as residual moisture, lighting and/or radiation.

In variants to the embodiments disclosed above, the estimators are different and/or displayed differently, for instance the display of the aggregated likelihood estimator is replaced by the display of the physico-chemical parameter likelihood estimator.

**Claims**

1. Method for determining at a current time point a preservation state of one product (11) of a group (10) of products made in a batch according to a predefined specification, each product (11) of said group (10) of products having a quantitative attribute taking a value evolving as a function of time and temperature, said preservation state of said one product (11) depending on said quantitative attribute value of said one product (11) at said current time point, said method including:

> (i) the step of determining a law of evolution - as a function of time - of said quantitative attribute value, including the step of receiving an experimental stability data set by conducting experiments in which a plurality of reference products for said group (10) of products is stored at a constant temperature within predefined tolerances and in which at a plurality of different experimental time points - including an experimental initial time point - respective quantitative attribute values of respective reference products are determined and recorded; and including the step of extrapolating said law of evolution from said experimental data set;
> (ii) the step of determining at said current time point the quantitative attribute value given by the law of evolution determined at step (i), whereby said current time point can be different from said experimental time points; and
> (iii) the step of determining said preservation state of said one product at said current time point using the quantitative attribute value determined at step (ii);

wherein:

> said method further includes the step of providing a computer system (15) in which is stored a plurality of models of evolution of said quantitative attribute value as a function of time and temperature, each said model having between three and nine parameters, said parameters including a quantitative attribute value at an initial time point, hereinafter termed initial quantitative attribute value parameter, said computer system (15) including an interface (16) for entering said experimental stability data set, including an equation resolution tool (18) for computing a previously entered experimental stability data set for finding for each said model best fitting values for its parameters, and including an estimator production tool (19) for finding predetermined estimators for each model, said estimators including a physico-chemical parameter likelihood estimator, a Bayesian Information Criterion, a Fischer test, a t-test and a fit distance, said physico-chemical parameter likelihood estimator being based at least on a comparison between initial quantitative attribute values in the experimental data set and the best fitting value found for said each model for the initial quantitative attribute value parameter, said fit distance being based on a comparison between the values in the experimental data set and the corresponding values given by said each model;
> in said step of producing an experimental stability data set, said plurality of reference products is stored at least at two different predetermined constant temperatures within predefined tolerances;

> - said step of extrapolating said law of evolution from said experimental stability data set includes the following steps:

>> a) the step of receiving said experimental stability data set into said computer system (15) through said interface (16);
>> b) the step of computing said experimental stability data set with said equation resolution tool (18) in said computer system (15) and the step of recording the found best fitting values for the parameters of each said model;
>> c) the step of further computing said experimental stability data set with said estimator production tool (19) in said computer system (15) and the step of recording the found estimators for each model; and
>> d) the step of selecting a best model amongst said models based on the respective found estimators, whereby the determined law of evolution of the quantitative attribute value is the selected best model with the found best fitting values for its parameters; **characterized in that**:

>>> step (ii) is carried out by calculation using successive temperature values taken by said one product (11) as from an initial time point up to said current time point by entering into the computer system (15) through the interface (16) the time and a temperature profile over the time, the quantitative value calculation tool (28) determining the quantitative attribute value at the current time point by successive iterations, that is first determining the quantitative attribute value after a first temperature step of the temperature profile at the beginning of the temperature profile, in which the temperature is constant,

starting with the quantitative attribute value at the beginning of the temperature profile, then iteratively determining the quantitative attribute value after each other temperature step of the temperature profile in which the temperature is constant, starting with the quantitative attribute value at the end of the previous temperature step, until the current time point,

step (i) further includes a determination of a confidence interval for the quantitative attribute values calculated with the determined law of evolution,

step (iii) includes the step of determining if the confidence interval for the value calculated at the current time point includes a predetermined rejection threshold value;

and

the step of selecting a best model selects a model that enables to find significant values for the confidence intervals of the parameters, and if at least two models enable to find significant values for the confidence intervals of the parameters, the step of selecting a best model selects the model meeting all the following criteria:

- the model for which the aggregated likelihood estimator is positive, the aggregated likelihood estimator being positive except

if at least one of the physico-chemical likelihood estimator, the Fisher test and the t-test is negative,

if the difference between the found initial quantitative attribute value parameter and the initial experimental quantitative attribute value is greater than a predetermined threshold or if the found initial quantitative attribute value parameter is negative,

- the model having a found initial quantitative attribute value parameter closest to the quantitative attribute value of the product at the initial time point,
- the model having the lowest fit distance, and
- the model having the lowest Bayesian Information Criterion if the number of parameters is the same for two models.

2. Method according to claim 1, further including the step of determining a modified experimental stability data set, the step of providing the modified experimental data set and the step of carrying out again step (i) to step (iii) with said modified experimental stability data set, wherein the modified experimental stability data set is the original experimental data set minus experimental data excluded as non-significant, for instance because one of the predetermined constant temperature is too high.

3. Method according to claim 1 or claim 2, wherein said parameters further include at least one of a parameter representative of an activation energy Ea and a parameter representative of a pre-exponential factor A and said physico-chemical likelihood parameter estimator is negative if Ea is less than 50 kJ/mol or greater than 1000 kJ/mol or if A is less than 1 kJ/mol or greater than 300 kJ/mol.

4. Method according to claim 3, wherein said parameters further include at least one parameter representative of an order of reaction and said physico chemical likelihood parameter estimator is negative if said at least one parameter representative of an order of reaction is less than 0 or greater than 10.

5. Method according to claim 3 or claim 4, wherein said parameters further include a parameter a representative of a proportion of subpopulation or representative of a fraction value at an initial time and said physico-chemical likelihood estimator is negative if a is less than 0 or greater than 1.

6. Method according to any of claims 1 to 5, wherein said estimators further include a quantitative statistics estimator based on at least one of a Fisher test and a t-test.

7. Method according to claim 6, wherein said step of providing a computer system (15) includes selecting said computer system as having a user interface displaying an aggregated likelihood estimator which is negative if said physico chemical likelihood estimator is negative or said quantitative statistics estimator is negative.

8. Method according to any of claims 1 to 7, wherein said step of providing a computer system (15) includes selecting said computer system as having a user interface displaying visual indications representative of said estimators found by said estimator production tool (19) and wherein said step of selecting a best model amongst said models is carried out by a user taking into account said visual indications representative of said estimators found by said estimator production tool (19).

9. Method according to any of claims 1 to 8, wherein each said model has between three and eight parameters.

10. Method according to any of claims 1 to 9, wherein said step (ii) is carried out by calculation using successive temperature values taken by said one product (11) as from an initial time point up to said current time point by entering into the computer system (15) through the interface (16) the time and a temperature profile over the time, the quantitative value calculation tool (28) determining the quantitative attribute value at the current time point by successive iterations, that is first determining the quantitative attribute value after a first temperature step of the temperature profile at the beginning of the temperature profile, in which the temperature is constant, starting with the quantitative attribute value at the beginning of the temperature profile, then iteratively determining the quantitative attribute value after each other temperature step of the temperature profile in which the temperature is constant, starting with the quantitative attribute value at the end of the previous temperature step, until the current time point.

11. Method according to any of claims 1 to 10, wherein said current time point is the present moment.

12. Method according to claim 11, wherein, said steps (ii) and (iii) are carried out by an electronic time temperature indicator (eTTI).

13. Method according to any of claims 1 to 10, wherein said current time point is a future moment.

14. Computer system as specified in claim 1, wherein the computer system is further configured to perform the method of claim 1.


**Patentansprüche**

1. Verfahren zum Bestimmen, zu einem aktuellen Zeitpunkt, eines Konservierungszustands eines Produkts (11) einer Gruppe (10) von Produkten, die in einer Charge hergestellt werden, gemäß einer vordefinierten Spezifikation, wobei jedes Produkt (11) der Gruppe (10) von Produkten ein quantitatives Attribut aufweist, das einen Wert annimmt, der sich als Funktion von Zeit und Temperatur entwickelt, wobei der Konservierungszustand des einen Produkts (11) vom quantitativen Attributwert des einen Produkts (11) zum aktuellen Zeitpunkt abhängt, wobei das Verfahren Folgendes beinhaltet:

(i) den Schritt des Bestimmens eines Entwicklungsgesetzes - als Funktion der Zeit - des quantitativen Attributwerts, der den Schritt des Empfangens eines Versuchs-Stabilitätsdatensatzes durch Durchführen von Versuchen beinhaltet, bei denen eine Vielzahl von Referenzprodukten für die Gruppe (10) von Produkten bei einer konstanten Temperatur innerhalb vordefinierter Toleranzen gelagert werden und bei denen zu einer Vielzahl von unterschiedlichen Versuchszeitpunkten - die einen Versuchs-Anfangszeitpunkt beinhalten - jeweilige quantitative Attributwerte jeweiliger Referenzprodukte bestimmt und aufgezeichnet werden; und den Schritt des Extrapolierens des Entwicklungsgesetzes aus dem Versuchsdatensatz beinhaltet;
(ii) den Schritt des Bestimmens, zum aktuellen Zeitpunkt, des quantitativen Attributwerts, der durch das in Schritt (i) bestimmte Entwicklungsgesetz gegeben ist, wobei sich der aktuelle Zeitpunkt von den Versuchszeitpunkten unterscheiden kann; und
(iii) den Schritt des Bestimmens des Konservierungszustands des einen Produkts zum aktuellen Zeitpunkt unter Verwendung des in Schritt (ii) bestimmten quantitativen Attributwerts;

wobei:

das Verfahren weiter den Schritt des Bereitstellens eines Computersystems (15) beinhaltet, in dem eine Vielzahl von Entwicklungsmodellen des quantitativen Attributwerts als Funktion von Zeit und Temperatur gespeichert sind, wobei jedes Modell zwischen drei und neun Parameter aufweist, wobei die Parameter einen quantitativen Attributwert zu einem anfänglichen Zeitpunkt beinhalten, der nachstehend als anfänglicher quantitativer Attributwertparameter bezeichnet wird, wobei das Computersystem (15) eine Oberfläche (16) zum Eingeben des Versuchs-Stabilitätsdatensatzes beinhaltet, die ein Gleichungslösungstool (18) zum Berechnen eines zuvor eingegebenen Versuchs-Stabilitätsdatensatzes zum Finden von am besten passenden Werten für jedes Modell für dessen Parameter beinhaltet, und ein Schätzer-Produktionstool (19) zum Finden von vorbestimmten Schätzern für jedes Modell beinhaltet, wobei die Schätzer einen Schätzer der Wahrscheinlichkeit eines physikalisch-chemischen Parameters, ein Bayessches Informationskriterium, einen Fischer-Test, einen t-Test und einen Passabstand beinhalten, wobei der Schätzer der Wahrscheinlichkeit eines physikalisch-chemischen Parameters mindestens auf einem Vergleich zwischen anfänglichen quantitativen Attributwerten im Versuchsdatensatz und dem am besten passenden Wert, der für jedes Modell für den anfänglichen quantitativen Attributwertparameter gefunden wurde, basiert, wobei der Passabstand auf einem Vergleich zwischen den Werten im Versuchsdatensatz und den entsprechenden Werten, die durch jedes Modell gegeben sind, basiert; in dem Schritt des Produzierens eines Versuchs-Stabilitätsdatensatzes die Vielzahl von Referenzprodukten bei mindestens zwei unterschiedlichen vorbestimmten konstanten Temperaturen innerhalb vordefinierter Toleranzen gelagert werden;

- der Schritt des Extrapolierens des Entwicklungsgesetzes aus dem Versuchs-Stabilitätsdatensatz die folgenden Schritte beinhaltet:

a) den Schritt des Empfangens des Versuchs-Stabilitätsdatensatzes im Computersystem (15) über die Oberfläche (16);
b) den Schritt des Berechnens des Versuchs-Stabilitätsdatensatzes mit dem Gleichungslösungstool (18) in dem Computersystem (15) und den Schritt des Aufzeichnens der gefundenen, am besten passenden Werte für die Parameter jedes Modells;
c) den Schritt des weiteren Berechnens des Versuchs-Stabilitätsdatensatzes mit dem Schätzer-Produktionswerkzeug (19) in dem Computersystem (15) und den Schritt des Aufzeichnens der gefundenen Schätzer für jedes Modell; und
d) den Schritt des Auswählens eines besten Modells unter den Modellen auf Basis der jeweiligen gefundenen Schätzer, wobei das bestimmte Entwicklungsgesetz des quantitativen Attributwerts das ausgewählte beste Modell mit den gefundenen, am besten passenden Werten für dessen Parameter ist;
**dadurch gekennzeichnet, dass**:

Schritt (ii) durch Berechnung, unter Verwendung von aufeinanderfolgenden Temperaturwerten, die von dem einen Produkt (11) ab einem anfänglichen Zeitpunkt bis zu dem aktuellen Zeitpunkt durch Eingeben über die Oberfläche (16) in das Computersystem (15) erfasst werden, der Zeit und eines Temperaturprofils über der Zeit ausgeführt wird, wobei das Tool zur Berechnung des quantitativen Werts (28) den quantitativen Attributwert zu dem aktuellen Zeitpunkt durch aufeinanderfolgende Iterationen bestimmt, das heißt zuerst Bestimmen des quantitativen Attributwerts nach einem ersten Temperaturschritt des Temperaturprofils an dem Beginn des Temperaturprofils, in dem die Temperatur konstant ist, beginnend mit dem quantitativen Attributwert an dem Beginn des Temperaturprofils, dann iteratives Bestimmen des quantitativen Attributwerts nach jedem weiteren Temperaturschritt des Temperaturprofils, in dem die Temperatur konstant ist, beginnend mit dem quantitativen Attributwert an dem Ende des vorherigen Temperaturschritts, bis zum aktuellen Zeitpunkt,
Schritt (i) weiter eine Bestimmung eines Konfidenzintervalls für die quantitativen Attributwerte, die mit dem bestimmten Entwicklungsgesetz berechnet wurden, beinhaltet, Schritt (iii) den Schritt des Bestimmens beinhaltet, ob das Konfidenzintervall für den Wert, der zum aktuellen Zeitpunkt berechnet wurde, einen vorbestimmten Ablehnungsschwellenwert beinhaltet; und
der Schritt des Auswählens eines besten Modells ein Modell auswählt, das es ermöglicht, signifikante Werte für die Konfidenzintervalle der Parameter zu finden, und wenn mindestens zwei Modelle es ermöglichen, signifikante Werte für die Konfidenzintervalle der Parameter zu finden, der Schritt des Auswählens eines besten Modells das Modell auswählt, das alle folgenden Kriterien erfüllt:

- das Modell, bei dem der Schätzer der aggregierten Wahrscheinlichkeit positiv ist, wobei der Schätzer der aggregierten Wahrscheinlichkeit positiv ist, außer

wenn mindestens einer des Schätzers der physikalisch-chemischen Wahrscheinlichkeit, des Fisher-Tests und des t-Tests negativ ist,
wenn die Differenz zwischen dem gefundenen anfänglichen quantitativen Attributwertparameter und dem anfänglichen quantitativen Attributwert im Versuch größer ist als eine vorbestimmte Schwelle, oder

wenn der gefundene anfängliche quantitative Attributwert-Parameter negativ ist,

- das Modell, das einen gefundenen anfänglichen quantitativen Attributwert-Parameter aufweist, der dem quantitativen Attributwert des Produkts zu dem anfänglichen Zeitpunkt am nächsten kommt,
- das Modell, das den kleinsten Passabstand aufweist, und
- das Modell, das das kleinste Bayessche Informationskriterium aufweist, wenn die Anzahl an Parametern bei zwei Modellen gleich ist.

2. Verfahren nach Anspruch 1, das weiter den Schritt des Bestimmens eines modifizierten Versuchs-Stabilitätsdatensatzes, den Schritt des Bereitstellens des modifizierten Versuchsdatensatzes und den Schritt des erneuten Ausführens von Schritt (i) bis Schritt (iii) mit dem modifizierten Versuchs-Stabilitätsdatensatz beinhaltet, wobei der modifizierte Versuchs-Stabilitätsdatensatz der ursprüngliche Versuchsdatensatz abzüglich Versuchsdaten ist, die als nicht signifikant ausgeschlossen wurden, beispielsweise weil eine der vorbestimmten konstanten Temperaturen zu hoch ist.

3. Verfahren nach Anspruch 1 oder Anspruch 2, wobei die Parameter weiter mindestens einen von einem Parameter, der für eine Aktivierungsenergie Ea repräsentativ ist, und einem Parameter beinhalten, der für einen präexponentiellen Faktor A repräsentativ ist, und der Schätzer des physikalisch-chemischen Wahrscheinlichkeitsparameters negativ ist, wenn Ea kleiner als 50 kJ/mol oder größer als 1000 kJ/mol ist, oder wenn A kleiner als 1 kJ/mol oder größer als 300 kJ/mol ist.

4. Verfahren nach Anspruch 3, wobei die Parameter weiter mindestens einen Parameter beinhalten, der für eine Reaktionsreihenfolge repräsentativ ist, und der Schätzer des physikalisch-chemischen Wahrscheinlichkeitsparameters negativ ist, wenn der mindestens eine Parameter, der für eine Reaktionsreihenfolge repräsentativ ist, kleiner als 0 oder größer als 10 ist.

5. Verfahren nach Anspruch 3 oder Anspruch 4, wobei die Parameter weiter einen Parameter $\alpha$ beinhalten, der für einen Anteil der Unterpopulation repräsentativ ist oder für einen Fraktionswert zu einem anfänglichen Zeitpunkt repräsentativ ist, und der Schätzer der physikalisch-chemischen Wahrscheinlichkeit negativ ist, wenn $\alpha$ kleiner als 0 oder größer als 1 ist.

6. Verfahren nach einem der Ansprüche 1 bis 5, wobei die Schätzer weiter einen Schätzer einer quantitativen Statistik auf Basis mindestens eines von einem Fisher-Test und einem t-Test beinhalten.

7. Verfahren nach Anspruch 6, wobei der Schritt des Bereitstellens eines Computersystems (15) das Auswählen des Computersystems als eine Benutzeroberfläche aufweisend beinhaltet, die einen Schätzer der aggregierten Wahrscheinlichkeit anzeigt, der negativ ist, wenn der Schätzer der physikalisch-chemischen Wahrscheinlichkeit negativ ist oder der Schätzer der quantitativen Statistik negativ ist.

8. Verfahren nach einem der Ansprüche 1 bis 7, wobei der Schritt des Bereitstellens eines Computersystems (15) das Auswählen des Computersystems als eine Benutzeroberfläche aufweisend beinhaltet, die visuelle Hinweise anzeigt, die für die von dem Schätzer-Produktionswerkzeug (19) gefundenen Schätzer repräsentativ sind, und wobei der Schritt des Auswählens eines besten Modells unter den Modellen von einem Benutzer unter Berücksichtigung der visuellen Hinweise, die für die von dem Schätzer-Produktionswerkzeug (19) gefundenen Schätzer repräsentativ sind, ausgeführt wird.

9. Verfahren nach einem der Ansprüche 1 bis 8, wobei jedes Modell zwischen drei und acht Parameter aufweist.

10. Verfahren nach einem der Ansprüche 1 bis 9, wobei Schritt (ii) durch Berechnung, unter Verwendung von aufeinanderfolgenden Temperaturwerten, die von dem einen Produkt (11) ab einem anfänglichen Zeitpunkt bis zum aktuellen Zeitpunkt durch Eingeben über die Oberfläche (16) in das Computersystem (15) erfasst werden, der Zeit und eines Temperaturprofils über der Zeit ausgeführt wird, wobei das Tool zur Berechnung des quantitativen Werts (28) den quantitativen Attributwert zum aktuellen Zeitpunkt durch aufeinanderfolgende Iterationen bestimmt, das heißt zuerst Bestimmen des quantitativen Attributwerts nach einem ersten Temperaturschritt des Temperaturprofils an dem Beginn des Temperaturprofils, in dem die Temperatur konstant ist, beginnend mit dem quantitativen Attributwert an dem Beginn des Temperaturprofils, dann iteratives Bestimmen des quantitativen Attributwerts nach jedem weiteren Temperaturschritt des Temperaturprofils, in dem die Temperatur konstant ist, beginnend mit dem quantitativen Attributwert an dem Ende des vorherigen Temperaturschritts, bis zu dem aktuellen Zeitpunkt.

11. Verfahren nach einem der Ansprüche 1 bis 10, wobei der aktuelle Zeitpunkt der gegenwärtige Moment ist.

12. Verfahren nach Anspruch 11, wobei die Schritte (ii) und (iii) von einem elektronischen Zeit-Temperatur-Indikator (eTTI) ausgeführt werden.

13. Verfahren nach einem der Ansprüche 1 bis 10, wobei der aktuelle Zeitpunkt ein zukünftiger Moment ist.

14. Computersystem nach Anspruch 1, wobei das Computersystem weiter so konfiguriert ist, dass es das Verfahren nach Anspruch 1 durchführt.


**Revendications**

1. Procédé pour la détermination, à un instant actuel, d'un état de conservation d'un produit (11) d'un groupe (10) de produits fabriqués en lot selon une spécification prédéfinie, chaque produit (11) dudit groupe (10) de produits ayant un attribut quantitatif prenant une valeur qui évolue en fonction du temps et de la température, ledit état de conservation dudit un produit (11) dépendant de ladite valeur d'attribut quantitatif dudit un produit (11) audit instant actuel, ledit procédé incluant :

   (i) l'étape de détermination d'une loi d'évolution - en fonction du temps - de ladite valeur d'attribut quantitatif, incluant l'étape de réception d'un jeu de données expérimentales de stabilité par la conduite d'expériences dans lesquelles une pluralité de produits de référence pour ledit groupe (10) de produits est stockée à une température constante au sein de tolérances prédéfinies et dans lesquelles à une pluralité d'instants expérimentaux différents - dont un instant expérimental initial - des valeurs d'attribut quantitatif respectives de produits de référence respectifs sont déterminées et enregistrées ; et incluant l'étape d'extrapolation de ladite loi d'évolution à partir dudit jeu de données expérimentales ;
   (ii) l'étape de détermination, audit instant actuel, de la valeur d'attribut quantitatif donnée par la loi d'évolution déterminée à l'étape (i), ledit instant actuel pouvant être différent desdits instants expérimentaux ; et
   (iii) l'étape de détermination dudit état de conservation dudit un produit audit instant actuel à l'aide de la valeur d'attribut quantitatif déterminée à l'étape (ii) ;

   dans lequel :

   ledit procédé inclut en outre l'étape de fourniture d'un système informatique (15) dans lequel est stockée une pluralité de modèles d'évolution de ladite valeur d'attribut quantitatif en fonction du temps et de la température, chaque dit modèle ayant entre trois et neuf paramètres, lesdits paramètres incluant une valeur d'attribut quantitatif à un instant initial, ci-après dénommé paramètre de valeur initiale d'attribut quantitatif, ledit système informatique (15) incluant une interface (16) pour la saisie dudit jeu de données expérimentales de stabilité, incluant un outil de résolution d'équation (18) pour le calcul informatique d'un jeu de données expérimentales de stabilité précédemment saisi pour l'identification, pour chaque dit modèle, de meilleures valeurs d'ajustement pour ses paramètres, et incluant un outil de production d'estimateur (19) pour l'identification d'estimateurs prédéterminés pour chaque modèle, lesdits estimateurs incluant un estimateur de probabilité de paramètre physico-chimique, un critère d'information bayésien, un test de Fischer, un test t et une distance d'ajustement, ledit estimateur de probabilité de paramètre physico-chimique étant basé au moins sur une comparaison entre des valeurs initiales d'attribut quantitatif dans le jeu de données expérimentales et la meilleure valeur d'ajustement identifiée pour chaque dit modèle pour le paramètre de valeur initiale d'attribut quantitatif, ladite distance d'ajustement étant basée sur une comparaison entre les valeurs dans le jeu de données expérimentales et les valeurs correspondantes données par chaque dit modèle ;
   à ladite étape de production d'un jeu de données expérimentales de stabilité, ladite pluralité de produits de référence est stockée au moins à deux températures constantes prédéterminées différentes au sein de tolérances prédéfinies ;

      - ladite étape d'extrapolation de ladite loi d'évolution à partir dudit jeu de données expérimentales de stabilité inclut les étapes suivantes :

         a) l'étape de réception dudit jeu de données expérimentales de stabilité dans ledit système informatique (15) par le biais de ladite interface (16) ;

b) l'étape de calcul informatique dudit jeu de données expérimentales de stabilité avec ledit outil de résolution d'équation (18) dans ledit système informatique (15) et l'étape d'enregistrement des meilleures valeurs d'ajustement identifiées pour les paramètres de chaque dit modèle ;

c) l'étape de calcul informatique supplémentaire dudit jeu de données expérimentales de stabilité avec ledit outil de production d'estimateur (19) dans ledit système informatique (15) et l'étape d'enregistrement des estimateurs identifiés pour chaque modèle ; et

d) l'étape de sélection d'un meilleur modèle parmi lesdits modèles sur la base des estimateurs identifiés respectifs, la loi d'évolution déterminée de la valeur d'attribut quantitatif étant le meilleur modèle sélectionné doté des meilleures valeurs d'ajustement identifiées pour ses paramètres ; **caractérisé en ce que** :

l'étape (ii) est réalisée par calcul à l'aide de valeurs de température successives prises par ledit un produit (11) d'un instant initial jusqu'audit instant actuel par la saisie, dans le système informatique (15), par le biais de l'interface (16), du temps et d'un profil de température au cours du temps, l'outil de calcul de valeur quantitative (28) déterminant la valeur d'attribut quantitatif à l'instant actuel par itérations successives, à savoir d'abord la détermination de la valeur d'attribut quantitatif après un premier pas de température du profil de température au début du profil de température, dans lequel la température est constante, en commençant par la valeur d'attribut quantitatif au début du profil de température, puis la détermination, de manière itérative, de la valeur d'attribut quantitatif après chaque autre pas de température du profil de température dans lequel la température est constante, en commençant par la valeur d'attribut quantitatif à la fin du pas de température précédent, jusqu'à l'instant actuel,

l'étape (i) inclut en outre une détermination d'un intervalle de confiance pour les valeurs d'attribut quantitatif calculées avec la loi d'évolution déterminée, l'étape (iii) inclut l'étape de détermination de si l'intervalle de confiance pour la valeur calculée à l'instant actuel inclut une valeur seuil de rejet prédéterminée ; et

l'étape de sélection d'un meilleur modèle sélectionne un modèle qui permet d'identifier des valeurs significatives pour les intervalles de confiance des paramètres, et si au moins deux modèles permettent d'identifier des valeurs significatives pour les intervalles de confiance des paramètres, l'étape de sélection d'un meilleur modèle sélectionne le modèle satisfaisant tous les critères suivants :

- le modèle pour lequel l'estimateur de probabilité agrégé est positif, l'estimateur de probabilité agrégé étant positif sauf

si au moins l'un parmi l'estimateur de probabilité physico-chimique, le test de Fisher et le test t est négatif,

si la différence entre le paramètre de valeur initiale d'attribut quantitatif identifié et la valeur initiale d'attribut quantitatif expérimentale est supérieure à un seuil prédéterminé ou

si le paramètre de valeur initiale d'attribut quantitatif identifié est négatif,

- le modèle ayant un paramètre de valeur initiale d'attribut quantitatif identifié le plus proche de la valeur d'attribut quantitatif du produit à l'instant initial,

- le modèle ayant la distance d'ajustement la plus faible, et

- le modèle ayant le critère d'information bayésien le plus faible si le nombre de paramètres est le même pour deux modèles.

2. Procédé selon la revendication 1, incluant en outre l'étape de détermination d'un jeu de données expérimentales de stabilité modifié, l'étape de fourniture du jeu de données expérimentales modifié et l'étape de réalisation, à nouveau, de l'étape (i) à l'étape (iii) avec ledit jeu de données expérimentales de stabilité modifié, dans lequel le jeu de données expérimentales de stabilité modifié est le jeu de données expérimentales d'origine moins des données expérimentales exclues comme étant non significatives, par exemple car l'une des températures constantes prédéterminées est trop élevée.

3. Procédé selon la revendication 1 ou la revendication 2, dans lequel lesdits paramètres incluent en outre au moins l'un parmi un paramètre représentatif d'une énergie d'activation Ea et un paramètre représentatif d'un facteur pré-exponentiel A et ledit estimateur de paramètre de probabilité physico-chimique est négatif si Ea est inférieur à 50 kJ/mol ou supérieur à 1000 kJ/mol ou si A est inférieur à 1 kJ/mol ou supérieur à 300 kJ/mol.

4. Procédé selon la revendication 3, dans lequel lesdits paramètres incluent en outre au moins un paramètre représentatif d'un ordre de réaction et ledit estimateur de paramètre de probabilité physico-chimique est négatif si ledit au moins un paramètre représentatif d'un ordre de réaction est inférieur à 0 ou supérieur à 10.

5. Procédé selon la revendication 3 ou la revendication 4, dans lequel lesdits paramètres incluent en outre un paramètre $\alpha$ représentatif d'une proportion de sous-population ou représentatif d'une valeur fractionnaire à un instant initial et ledit estimateur de probabilité physico-chimique est négatif si $\alpha$ est inférieur à 0 ou supérieur à 1.

6. Procédé selon l'une quelconque des revendications 1 à 5, dans lequel lesdits estimateurs incluent en outre un estimateur statistique quantitatif basé sur au moins l'un parmi un test de Fisher et un test t.

7. Procédé selon la revendication 6, dans lequel ladite étape de fourniture d'un système informatique (15) comporte la sélection dudit système informatique comme ayant une interface utilisateur affichant un estimateur de probabilité agrégé qui est négatif si ledit estimateur de probabilité physico-chimique est négatif ou si ledit estimateur statistique quantitatif est négatif.

8. Procédé selon l'une quelconque des revendications 1 à 7, dans lequel ladite étape de fourniture d'un système informatique (15) comporte la sélection dudit système informatique comme ayant une interface utilisateur affichant des indications visuelles représentatives desdits estimateurs identifiés par ledit outil de production d'estimateur (19) et dans lequel ladite étape de sélection d'un meilleur modèle parmi lesdits modèles est réalisée par un utilisateur prenant en compte lesdites indications visuelles représentatives desdits estimateurs identifiés par ledit outil de production d'estimateur (19).

9. Procédé selon l'une quelconque des revendications 1 à 8, dans lequel chaque dit modèle possède entre trois et huit paramètres.

10. Procédé selon l'une quelconque des revendications 1 à 9, dans lequel ladite étape (ii) est réalisée par calcul informatique à l'aide de valeurs de température successives prises par ledit un produit (11) d'un instant initial jusqu'audit instant actuel par la saisie, dans le système informatique (15), par le biais de l'interface (16), du temps et d'un profil de température au cours du temps, l'outil de calcul de valeur quantitative (28) déterminant la valeur d'attribut quantitatif à l'instant actuel par itérations successives, à savoir d'abord la détermination de la valeur d'attribut quantitatif après un premier pas de température du profil de température au début du profil de température, dans lequel la température est constante, en commençant par la valeur d'attribut quantitatif au début du profil de température, puis la détermination, de manière itérative, de la valeur d'attribut quantitatif après chaque autre pas de température du profil de température dans lequel la température est constante, en commençant par la valeur d'attribut quantitatif à la fin du pas de température précédent, jusqu'à l'instant actuel.

11. Procédé selon l'une quelconque des revendications 1 à 10, dans lequel ledit instant actuel est le moment présent.

12. Procédé selon la revendication 11, dans lequel lesdites étapes (ii) et (iii) sont réalisées par un indicateur temps-température électronique (eTTI).

13. Procédé selon l'une quelconque des revendications 1 à 10, dans lequel ledit instant actuel est un moment futur.

14. Système informatique selon la revendication 1, dans lequel le système informatique est en outre configuré pour réaliser le procédé selon la revendication 1.

EP 3 977 465 B1

Fig. 1

PRIOR ART

Fig. 2

PRIOR ART

DETERMINATION OF
LAW OF EVOLUTION

DETERMINATION OF
QUANTITATIVE ATTRIBUTE VALUE
AT CURRENT TIME POINT

DETERMINATION OF
PRESERVATION STATE
AT CURRENT TIME POINT

27

Fig. 3

Fig. 4

Fig. 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *AKTS®-Thermokinetics® software - Prediction of Shelf Life of Materials by Kinetic Analysis*, 02 May 2018, https: //www.youtube.com/watch?v=e2kPz0DgpXg